# EUROPEAN PATENT APPLICATION

(11) **EP 4 173 643 A1**
(43) Date of publication of application: **03.05.2023**
(21) Application number: 22212974.4
(22) Date of filing: 27.09.2021
(51) Int. Cl.: A61L 2/10, A61L 2/28

(54) **SYSTEMS AND METHODS FOR INDICATING ULTRAVIOLET SANITIZATION**

(30) Priority: 13.11.2020 US 202063113621 P
(62) Divisional of application: 21199241.7
(71) Applicant: The Boeing Company, Arlington, VA 22202 (US)
(72) Inventor: McIntosh, Darren C., Chicago, 60606-1596 (US)
(74) Representative: Witte, Weller & Partner Patentanwälte mbB

(57) **Abstract**

In an example, a protective assembly (100) for visually indicating ultraviolet (UV) sanitization of an area is described. The protective assembly (100) comprises a retrofitted protective bumper (102) attachable to one or more surfaces in the area such that the retrofitted protective bumper (102) covers at least a portion of the one or more surfaces. The retrofitted protective bumper (102) comprises a photochromic plastic material (106) that, when exposed to UV light, changes from a first color to a second color that is different from the first color and that is visible under ambient lighting conditions.

## Description

### BACKGROUND

Ultraviolet (UV) light is one type of existing solution for disinfecting or otherwise sanitizing surfaces within an aircraft, since UV light can often effectively disinfect exposed surfaces. After a surface has been sanitized (with UV light or other solution), it can be desirable to provide a visual indicator that makes passengers, crew members, or other individuals aware that the surface has been sanitized.

Existing solutions for indicating that surfaces have been sanitized include photoluminescent indicators. However, these existing solutions typically do not provide a visual indication that the sanitization has been performed to a satisfactory level. In addition, the visual indication provided by these existing solutions might not be visible without a black light or other visual aid. Further, some existing photoluminescent indicators can include materials that charge and glow when exposed to other light sources in addition to UV light. Thus, there can be situations in which cabin lighting or other forms of lighting present in the aircraft, other than UV light, can cause the existing photoluminescent indicators to glow even when the photoluminescent indicators were not exposed to UV light, thereby inaccurately indicating that a surface has been sanitized when that surface might not have been sanitized.

What is needed is a device that provides an easily-accessible, more-accurate indication of when a surface has been sanitized with UV light.

### SUMMARY

In an example, a protective assembly for visually indicating ultraviolet (UV) sanitization of an area is described. The protective assembly comprises a retrofitted protective bumper attachable to one or more surfaces in the area such that the retrofitted protective bumper covers at least a portion of the one or more surfaces. The retrofitted protective bumper comprises a photochromic plastic material that, when exposed to UV light, changes from a first color to a second color that is different from the first color and that is visible under ambient lighting conditions.

In another example, a method is described. The method comprises exposing a retrofitted protective bumper to ultraviolet (UV) light using a light-emitter device configured to emit the UV light, where the retrofitted protective bumper is attached to one or more surfaces in an area such that the retrofitted protective bumper covers at least a portion of the one or more surfaces, and where the retrofitted protective bumper comprises a photochromic plastic material having a first color. The method also comprises in response to the exposing, causing the photochromic plastic material to change from the first color to a second color that is different from the first color, that is visible under ambient lighting conditions, and that visually indicates UV sanitization of the area.

In another example, a method is described. The method comprises mechanically coupling, to a bracket attached to one or more surfaces in an area, a retrofitted protective bumper, such that the retrofitted protective bumper covers at least a portion of the one or more surfaces. The retrofitted protective bumper comprises a photochromic plastic material that, when exposed to ultraviolet (UV) light, changes from a first color to a second color that is different from the first color, that is visible under ambient lighting conditions, and that visually indicates UV sanitization of the area.

In another example, an ultraviolet (UV) sanitization kit is described. The UV sanitization kit comprises a light-emitter device configured to emit UV light. The UV sanitization kit also comprises a retrofitted protective bumper, where the retrofitted protective bumper is an insert configured to mechanically couple to a bracket attached to one or more surfaces in an area such that the retrofitted protective bumper covers at least a portion of the one or more surfaces. The retrofitted protective bumper comprises a photochromic plastic material that, when exposed to UV light, changes from a first color to a second color that is different from the first color and that is visible under ambient lighting conditions, thereby visually indicating UV sanitization of the area.

In another example, a protective assembly for visually indicating ultraviolet (UV) sanitization of an area. The protective assembly comprises a retrofitted protective bumper attachable to one or more surfaces in the area such that the retrofitted protective bumper covers at least a portion of the one or more surfaces. The retrofitted protective bumper comprises a photochromic plastic material that, when exposed to UV light, changes from a first color to a second color that is different from the first color. The photochromic plastic material comprises one or more photochromic polymers, present in a combined amount of at least 5 wt.%. The photochromic plastic material also comprises at least one of a colloidal metal, or a non-photochromic polymer.

The features, functions, and advantages that have been discussed can be achieved independently in various examples or may be combined in yet other examples. Further details of the examples can be seen with reference to the following description and drawings.

### BRIEF DESCRIPTION OF THE FIGURES

The novel features believed characteristic of the illustrative examples are set forth in the appended claims. The illustrative examples, however, as well as a preferred mode of use, further objectives and descriptions thereof, will best be understood by reference to the following detailed description of an illustrative example of the present disclosure when read in conjunction with the accompanying drawings, wherein:
Figure 1 depicts a protective assembly for visually indicating UV sanitization of an area, according to an example implementation.
Figure 2 depicts the protective assembly of Figure 1 after a photochromic plastic material of a retrofitted protective bumper has changed colors, according to an example implementation.
Figure 3 depicts the protective assembly of Figure 1, according to an example implementation.
Figure 4 depicts the protective assembly of Figure 1, according to an example implementation.
Figure 5 depicts the protective assembly of Figure 1, according to an example implementation.
Figure 6 depicts a perspective view of an aircraft, according to an example implementation.
Figure 7 shows a flowchart of an example method, according to an example implementation.
Figure 8 shows a flowchart of another example method, according to an example implementation.

### DETAILED DESCRIPTION

Disclosed examples will now be described more fully hereinafter with reference to the accompanying drawings, in which some, but not all of the disclosed examples are shown. Indeed, several different examples may be described and should not be construed as limited to the examples set forth herein. Rather, these examples are described so that this disclosure will be thorough and complete and will fully convey the scope of the disclosure to those skilled in the art.

By the terms "substantially," "about," "approximately," and "proximate" used herein, it is meant that the recited characteristic, parameter, or value need not be achieved exactly, but that deviations or variations, including for example, tolerances, measurement error, measurement accuracy limitations and other factors known to skill in the art, may occur in amounts that do not preclude the effect the characteristic was intended to provide.

Unless otherwise specifically noted, elements depicted in the drawings are not necessarily drawn to scale.

Within examples, described herein are systems and methods for visually indicating UV sanitization of an area, such as an area on an aircraft. In particular, the described systems and methods provide a retrofitted protective bumper that is attachable to one or more surfaces in the area such that the retrofitted protective bumper covers and protects at least a portion of the one or more surfaces. The retrofitted protective bumper comprises a photochromic plastic material that, when exposed to UV light, changes from a first color to a second color that is different from the first color. The second color is visible under ambient lighting conditions and can be visible under such ambient lighting conditions without a visual aid device (e.g., a black light source or other type of light source).

In this disclosure, examples are described primarily with respect to an aircraft. However, it should be understood that, in other implementations, the disclosed systems and methods can be implemented in vehicles other than an aircraft, such as an aerospace vehicle such as marine vehicle, rotocraft, space vehicle, or the like; transportation vehicles such as automobiles, busses, boats, ships, subways, trains, amusement rides, and the like; and/or can be implemented in other contexts and locations, such as in buildings including schools, theatres, commercial buildings, airports, and the like. The disclosed systems can also be used in outdoor areas such as amusement parks, amphitheaters, and the like.

The bumpers, rubstrips and the like can be prepared by extrusion, die cast, injection molding, or the like.

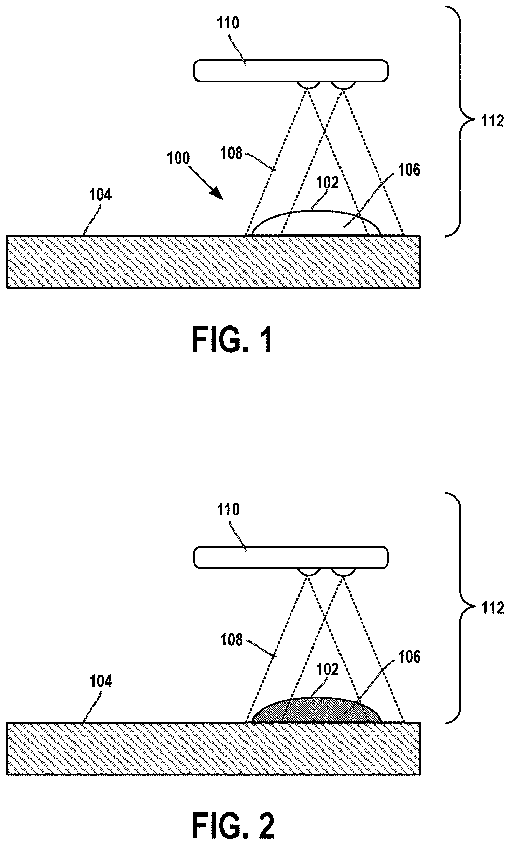

Referring now to the figures, Figure 1 depicts an example of a protective assembly 100 for visually indicating UV sanitization of an area. The protective assembly 100 includes a retrofitted protective bumper 102 that is attachable to one or more surfaces 104 in the area such that the retrofitted protective bumper 102 covers at least a portion of the one or more surfaces 104. In Figure 1, the retrofitted protective bumper 102 is shown to be attached to the one or more surfaces 104. Within examples, the retrofitted protective bumper 102 is a rubstrip, corner bumper, or other type of protective bumper, and the one or more surfaces 104 are located in areas of an aircraft (not shown) that are high-traffic areas (e.g., a main cabin that is often occupied by numerous passengers) or that include surfaces that are otherwise frequently exposed to humans or objects that touch, bump, or otherwise interact with the surfaces. For instance, the retrofitted protective bumper 102 is attached to the one or more surfaces 104 on a floor of an aisleway of the aircraft or on a corner in or near the lavatory of the aircraft. Other examples are possible as well.

To achieve the visual indication of UV sanitization of the area, the retrofitted protective bumper 102 includes a photochromic plastic material 106 that, when exposed to UV light 108, changes from a first color to a second color that is different from the first color and that is visible under ambient lighting conditions, thereby visually indicating UV sanitization of the area. Within examples, the photochromic plastic material 106 can be configured to change back from the second color to the first color after a predetermined period of time. Within examples, the predetermined period of time is 1 to 3 minutes, 2 to 5 minutes, or 4 to 7 minutes.

The visibility of the second color under ambient lighting conditions is an advantage over existing solutions for indicating sanitization, since a black light or other visual aid device might not, and in embodiments, is not needed in order to see the second color. Thus, after sanitizing the area with the UV light 108, an individual can quickly and easily see that the photochromic plastic material 106 of the retrofitted protective bumper 102 has changed from the first color to the second color.

Furthermore, in contrast to the photoluminescent indicators used with existing solutions for indicating sanitization, the photochromic plastic material 106 of the retrofitted protective bumper 102 can be less likely to change color when exposed to sunlight or other forms of lighting that might be present in an aircraft (e.g., cabin lighting), other vehicle, building, or other environment. Thus, the protective assembly 100 disclosed herein can reduce the chance of falsely indicating UV sanitization of an area.

Within examples, the photochromic plastic material 106 is a photochromic plastic formulation that makes up at least part of the retrofitted protective bumper 102, such as by adding a photochromic masterbatch to a non-photochromic plastic. For example, at least 50% of the retrofitted protective bumper 102 is made up of one or more photochromic polymers (e.g., High-density polyethylene (HDPE), Polypropylene (PP), and/or Thermoplastic Polyurethane (TPU)). More specifically, the photochromic polymer(s) make up from about 50 to about 100 %, or from about 75 to about 100%, or from about 85 to about 100% by weight of total solids of the photochromic plastic formulation. As another example, the photochromic plastic formulation includes the one or more photochromic polymers, present in a combined amount of at least 5 wt.%, and further includes a colloidal metal and/or a non-photochromic polymer, such as a non-photochromic plastic material. That is, a particular amount of photochromic polymer(s) is/are combined with a remainder of the materials that make up the retrofitted protective bumper 102. In embodiments, the photochromic plastic formulation includes photochromic polymer(s) in an amount of from about 5 to about 25, or from about 10 to about 30, or from about 15 to about 35 percent by weight of total solids of the photochromic plastic formulation. To facilitate this, for instance, the photochromic plastic material 106 can take the form of photochromic plastic pellets that are mixed into a melted composition that includes at least one non-photochromic plastic (e.g., polyvinyl polymers) and/or includes a colloidal metal, and the composition is then formed into the retrofitted protective bumper 102. Other examples are possible as well.

Within additional examples, the retrofitted protective bumper 102 includes a coating of the photochromic plastic material 106. For instance, the coating can be disposed adjacent to an outer surface of a non-photochromic base layer or structure (e.g., a non-photochromic plastic or a metal). The photochromic plastic material 106 can make up at least a threshold percentage of the coating, such as 50%. Further, the coating can be disposed adjacent to at least a threshold percentage of the outer surface of the non-photochromic base layer or structure, such as 95%-100% of the outer surface. In these and other examples described above, the photochromic plastic material 106 can be substantially homogeneous.

Within examples, the UV light 108 is selected to have a wavelength that is within a range of approximately 200 nanometers to about 280 nanometers, or from about 222 nanometers to about 280 nanometers, or from about 222 nanometers to about 254 nanometers, or from about 222 nanometers to approximately 240 nanometers, or from about 240 nanometers to about 280 nanometers.

Within examples, and as shown in Figure 1, a light-emitter device 110 is configured to emit the UV light 108. Figure 1 shows the photochromic plastic material 106 having the first color while the light-emitter device 110 has begun to expose the photochromic plastic material 106 to the UV light 108. The light-emitter device 110 can be a UV wand or other UV light source. As the light-emitter device 110 emits the UV light 108 in the area, pathogens can be destroyed, thereby sanitizing the area. In some scenarios, for instance, pathogens on a surface can be destroyed immediately after, or within a predetermined period of time (e.g., a few seconds to a few minutes) after, the light-emitter device 110 is moved (e.g., swept) over the surface.

Both the light-emitter device 110 and the retrofitted protective bumper 102 can be included in a UV sanitization kit 112. Within examples, the UV sanitization kit 112 includes other devices or features as well, such as one or more fasteners or other devices used to attach the retrofitted protective bumper 102 directly or indirectly to the one or more surfaces 104. Other examples are possible as well.

In embodiments, the retrofitted protective bumper 102, including but not limited to the photochromic plastic material 106 is designed/selected to meet one or more regulations associated with the aircraft, other vehicle, building, other indoor venue, or outdoor venue where the retrofitted protective bumper 102 is located. For example, the retrofitted protective bumper 102 is non-flammable (e.g., comprised of materials that do not ignite, burn, release flammable vapors, etc. when exposed to fire or heat) and/or otherwise must meet applicable test criteria for flammability as set forth by the Federal Aviation Administration (FAA).

Figure 2 next depicts the protective assembly 100 while/after the photochromic plastic material 106 has been exposed to the UV light 108 and after the first color has changed to the second color.

Within examples, the first color is selected to substantially match a color of an interior of an aircraft, other mode of transportation, or a building, or the first color is chosen for aesthetic value. For instance, the first color is selected to substantially match a color of a corner or aisleway of the aircraft, or a color is chosen for aesthetic value. Within other examples, the first color is a clear, transparent color, and the second color is red, blue, purple, or yellow, so as to sufficiently distinguish from the first color.

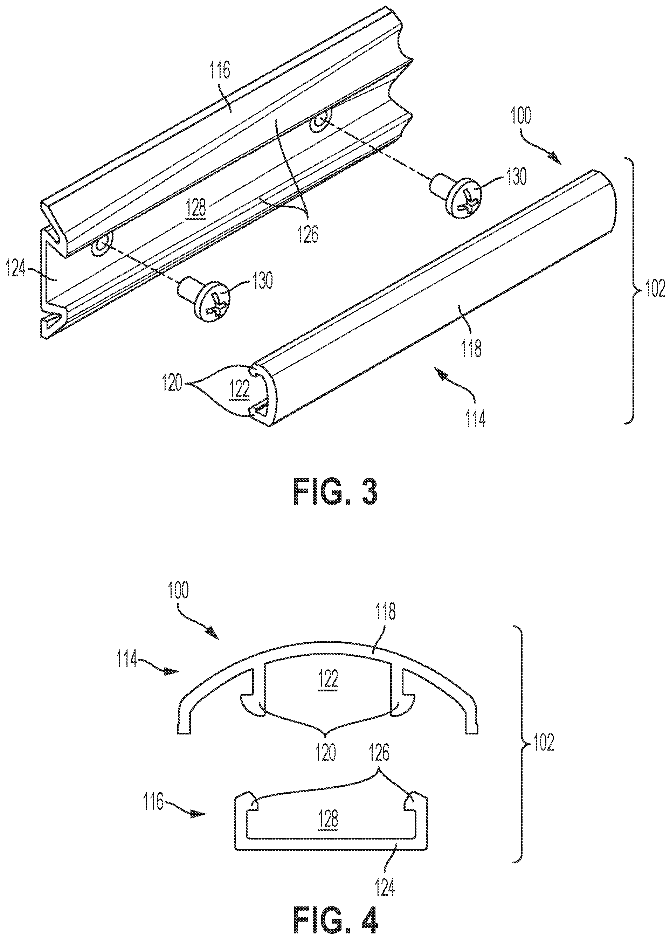

Figure 3 depicts a perspective view of an example of the protective assembly 100 in which the retrofitted protective bumper 102 includes an insert 114 that is configured to be mechanically coupled to a bracket 116 that is attached to the one or more surfaces 104 (not shown). More particularly, in the example shown in Figure 3, the retrofitted protective bumper 102 is the insert 114 and the insert 114 is configured to be mechanically coupled to a separate device - namely, the bracket 116. In other examples, however, such as the example shown in Figure 4, the retrofitted protective bumper 102 can include both the insert 114 and the bracket 116, where the insert 114 is mechanically coupled to the bracket 116. Before or after coupling the insert 114 to the bracket 116, the bracket 116 can be attached to the one or more surfaces 104. In still other examples where the retrofitted protective bumper 102 is an insert or equivalent structure, the retrofitted protective bumper 102 can be configured to mechanically couple (or couple in other manners) to another type of device, other than a bracket.

As shown in Figure 3, the insert 114 includes an elongated arcuate base 118 and a first pair of legs 120 protruding from the elongated arcuate base 118 and defining a first channel 122 therebetween. The elongated arcuate base 118 can take an arcuate or other substantially-rounded shape to desirably protect the one or more surfaces 104 and to desirably absorb contact. As further shown, the bracket 116 includes an elongated base 124 and a second pair of legs 126 protruding from the elongated base and defining a second channel 128 therebetween. The bracket 116 is attachable to the one or more surfaces 104 via one or more fasteners 130, such as screws or pins, in order to retain the bracket 116 against the one or more surfaces 104. For instance, the one or more fasteners 130 can take the form of a series of fasteners distributed along a length of the elongated base 124 within the second channel 128.

As further shown, the insert 114 is configured for snap-fit insertion into the second channel 128 of the bracket 116 such that the first pair of legs 120 interlock with the second pair of legs 126. In particular, each leg of the first pair of legs 120 and the second pair of legs 126 has a cantilever design for snap-fitting. Other snap-fit designs are possible as well.

Embodiments in which the retrofitted protective bumper 102 is the insert 114 are advantageous in situations in which an existing, non-photochromatic insert might already be attached to the bracket 116 because the insert 114 can then be used to replace the existing, non-photochromatic insert. The example structure of the insert 114 and the bracket 116 shown in Figure 3, as well as other possible example structures, can facilitate efficient replacement of existing, non-photochromatic inserts with retrofitted protective bumpers having the photochromic plastic material 106, such as the insert 114.

Figure 4 depicts an example of the protective assembly 100 in which the retrofitted protective bumper 102 includes the insert 114 and the bracket 116. As shown, the insert 114 and the bracket 116 include the respective components described above, but take different forms than those shown in Figure 3.

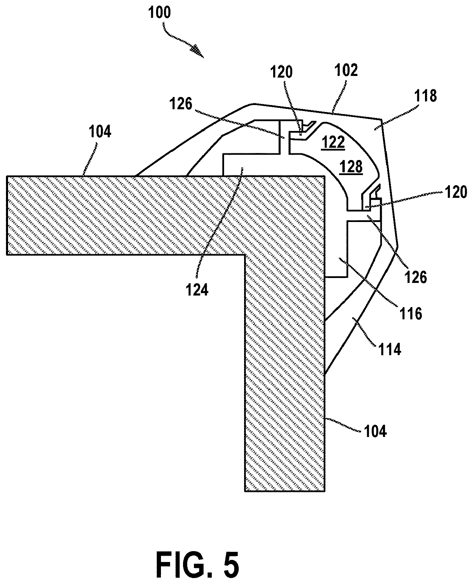

Figure 5 depicts a top-down view of another example of the protective assembly 100 in which the retrofitted protective bumper 102 includes the insert 114 that is configured to be mechanically coupled to the bracket 116. More specifically, the one or more surfaces 104 are surfaces of a corner (e.g., of a wall), and thus the retrofitted protective bumper 102 protects the corner and acts as a way to determine of the area that includes the corner has been UV sanitized. The bracket 116 is shown to be attached to the one or more surfaces 104.

It should be understood that the insert 114 and the bracket 116 described above can take other forms, with other shapes and dimensions.

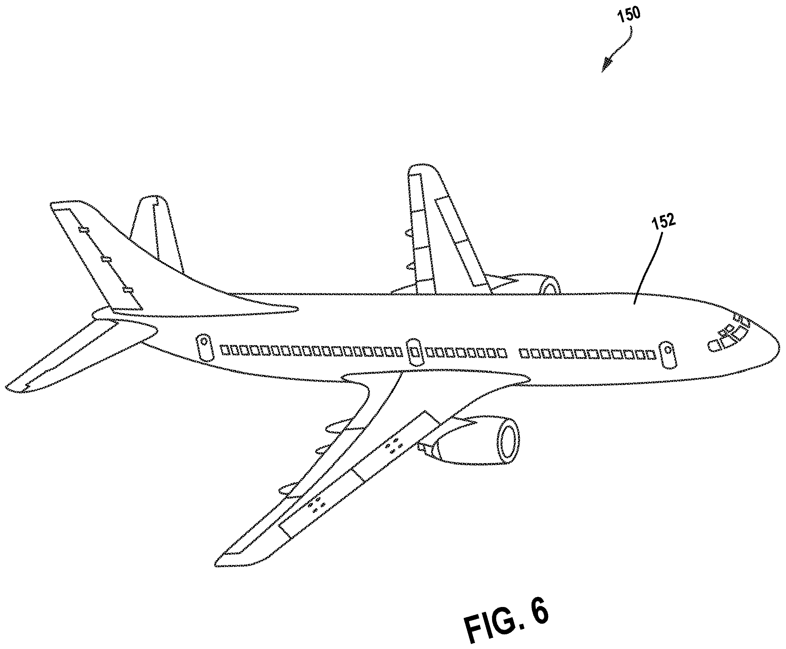

Figure 6 is a perspective view of an aircraft 150, according to an example implementation. As noted above, the retrofitted protective bumper 102 (not shown in Figure 6) can be attached to one or more surfaces 104 in an area within a fuselage 152 of the aircraft 150, such as in a main cabin of the aircraft 150.

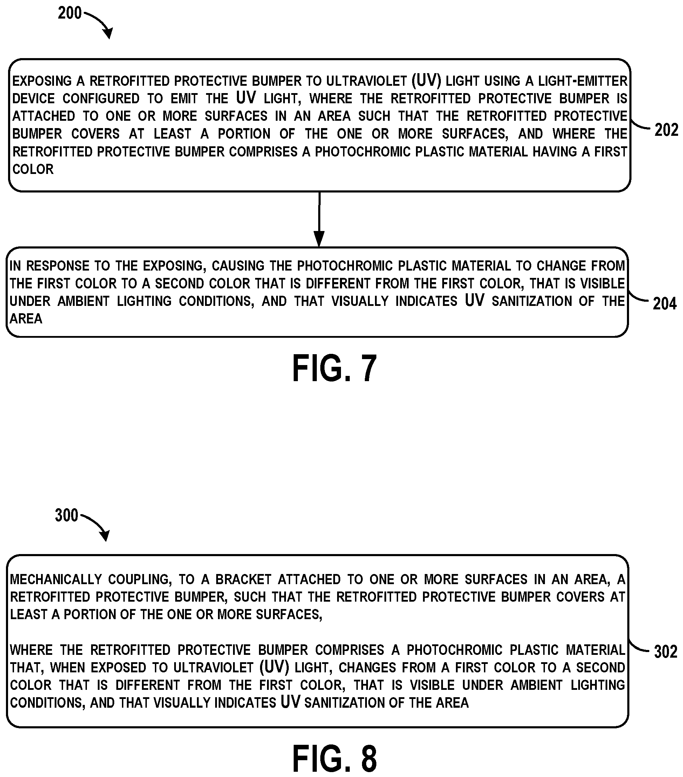

Figure 7 shows a flowchart of an example of a method 200 that could be used with the protective assembly 100 and UV sanitization kit 112 shown in Figures 1, 2, 3, 4, and 5. Method 200 may include one or more operations, functions, or actions as illustrated by one or more of blocks 202-204.

At block 202, the method 200 includes exposing a retrofitted protective bumper to UV light using a light-emitter device configured to emit the UV light, where the retrofitted protective bumper is attached to one or more surfaces in an area such that the retrofitted protective bumper covers at least a portion of the one or more surfaces, and wherein the retrofitted protective bumper comprises a photochromic plastic material having a first color.

At block 204, the method 200 includes in response to the exposing, causing the photochromic plastic material to change from the first color to a second color that is different from the first color, that is visible under ambient lighting conditions, and that visually indicates UV sanitization of the area.

Within examples, the exposing as shown in block 202 is performed by exposing the retrofitted protective bumper to UV light having a wavelength within a range of approximately 200 nanometers to approximately 280 nanometers.

Within examples, the exposing as shown in block 202 is performed by exposing the retrofitted protective bumper to UV light having a wavelength within a range of approximately 222 nanometers to approximately 254 nanometers. Other ranges are possible as well.

Within examples, the retrofitted protective bumper is attached to one or more surfaces in an aisleway or corner within an aircraft, and thus the exposing as shown in block 202 is performed by exposing, to the UV light, the retrofitted protective bumper that is attached to one or more surfaces in the aisleway or the corner within the aircraft.

Within examples, the causing as shown in block 204 is performed by causing the photochromic plastic material to change from the first color to a second color that is visible under the ambient lighting conditions without a visual aid device.

Within examples, the retrofitted protective bumper is non-flammable.

Figure 8 shows a flowchart of an example of a method 300 that could be used with the protective assembly 100 and UV sanitization kit 112 shown in Figures 1, 2, 3, 4, and 5. Method 300 may include one or more operations, functions, or actions as illustrated by block 302, although additional operations, functions, or actions are possible.

At block 302, the method 300 includes mechanically coupling, to a bracket attached to one or more surfaces in an area, a retrofitted protective bumper, such that the retrofitted protective bumper covers at least a portion of the one or more surfaces. The retrofitted protective bumper comprises a photochromic plastic material that, when exposed to UV light, changes from a first color to a second color that is different from the first color, that is visible under ambient lighting conditions, and that visually indicates UV sanitization of the area.

Within examples, the retrofitted protective bumper comprises an insert having an elongated arcuate base and a first pair of legs protruding from the elongated arcuate base, defining a first channel therebetween, and the bracket comprises an elongated base and a second pair of legs protruding from the elongated base and defining a second channel therebetween. Within such examples, the coupling as shown in block 302 is performed by mechanically coupling the insert to the bracket using snap-fit insertion of the insert into the second channel of the bracket such that the first pair of legs interlock with the second pair of legs.

Within examples, the coupling as shown in block 302 is performed by mechanically coupling the retrofitted protective bumper to the bracket attached to one or more surfaces in an aisleway or corner within an aircraft.

Devices or systems may be used or configured to perform logical functions presented in Figures 7 and 8. In some instances, components of the devices and/or systems may be configured to perform the functions such that the components are actually configured and structured (with hardware and/or software) to enable such performance. In other examples, components of the devices and/or systems may be arranged to be adapted to, capable of, or suited for performing the functions, such as when operated in a specific manner. Although blocks in Figures 7 and 8, are illustrated in a sequential order, these blocks may also be performed in parallel, and/or in a different order than those described herein. Also, the various blocks may be combined into fewer blocks, divided into additional blocks, and/or removed based upon the desired implementation.

It should be understood that for these and other processes and methods disclosed herein, flowcharts show functionality and operation of one possible implementation of present examples. In this regard, each block or portions of each block may represent a module, a segment, or a portion of program code, which includes one or more instructions executable by a processor for implementing specific logical functions or steps in the process. The program code may be stored on any type of computer readable medium or data storage, for example, such as a storage device including a disk or hard drive. Further, the program code can be encoded on a computer-readable storage media in a machine-readable format, or on other non-transitory media or articles of manufacture. The computer readable medium may include non-transitory computer readable medium or memory, for example, such as computer-readable media that stores data for short periods of time like register memory, processor cache and Random Access Memory (RAM). The computer readable medium may also include non-transitory media, such as secondary or persistent long term storage, like read only memory (ROM), optical or magnetic disks, compact-disc read only memory (CD-ROM), for example. The computer readable media may also be any other volatile or non-volatile storage systems. The computer readable medium may be considered a tangible computer readable storage medium, for example.

In addition, each block or portions of each block in Figures 7 and 8 may represent circuitry that is wired to perform the specific logical functions in the process. Alternative implementations are included within the scope of the examples of the present disclosure in which functions may be executed out of order from that shown or discussed, including substantially concurrent or in reverse order, depending on the functionality involved, as would be understood by those reasonably skilled in the art.

Different examples of the system(s), device(s), and method(s) disclosed herein include a variety of components, features, and functionalities. It should be understood that the various examples of the system(s), device(s), and method(s) disclosed herein may include any of the components, features, and functionalities of any of the other examples of the system(s), device(s), and method(s) disclosed herein in any combination or any sub-combination, and all of such possibilities are intended to be within the scope of the disclosure.

Further, the disclosure comprises embodiments according to the following Examples:
Example 1. A protective assembly (100) for visually indicating ultraviolet (UV) sanitization of an area, the protective assembly (100) comprising:
   a retrofitted protective bumper (102) attachable to one or more surfaces (104) in the area such that the retrofitted protective bumper (102) covers at least a portion of the one or more surfaces (104),
   wherein the retrofitted protective bumper (102) comprises a photochromic plastic material (106) that, when exposed to UV light (108), changes from a first color to a second color that is different from the first color and that is visible under ambient lighting conditions.
Example 2. The protective assembly (100) of Example 1, wherein the second color is visible under the ambient lighting conditions without a visual aid device.
Example 3. The protective assembly (100) of Examples 1 or 2, wherein the retrofitted protective bumper (102) is non-flammable.
Example 4. The protective assembly (100) of any of Examples 1 to 3, wherein the photochromic plastic material (106) is configured to change from the first color to the second color when exposed to UV light (108) having a wavelength within a range of approximately 200 nanometers to approximately 280 nanometers.
Example 5. The protective assembly (100) of any of Examples 1 to 4, wherein the photochromic plastic material (106) is configured to change from the first color to the second color when exposed to UV light (108) having a wavelength within a range of approximately 222 nanometers to approximately 254 nanometers.
Example 6. The protective assembly (100) of any of Examples 1 to 5, wherein the retrofitted protective bumper (102) further comprises a non-photochromic plastic material (106).
Example 7. The protective assembly (100) of any of Examples 1 to 6, wherein the retrofitted protective bumper (102) further comprises a colloidal metal.
Example 8. The protective assembly (100) of any of Examples 1 to 7, wherein the retrofitted protective bumper (102) comprises an insert (114) configured to be mechanically coupled to a bracket (116) that is attached to the one or more surfaces (104).
Example 9. The protective assembly (100) of Example 8, wherein the insert (114) comprises an elongated arcuate base (118) and a first pair of legs (120) protruding from the elongated arcuate base (118) and defining a first channel (122) therebetween, and
   wherein the bracket (116) comprises an elongated base (124) and a second pair of legs (126) protruding from the elongated base (124) and defining a second channel (128) therebetween.
Example 10. The protective assembly (100) of Example 9, wherein the insert (114) is configured for snap-fit insert (114)ion into the second channel (128) of the bracket (116) such that the first pair of legs (120) interlock with the second pair of legs (126).
Example 11. The protective assembly (100) of any of Examples 1 to 10, wherein the retrofitted protective bumper (102) comprises an insert (114) mechanically coupled to a bracket (116),
   wherein the bracket (116) is attachable to the one or more surfaces (104) via one or more fasteners (130) to retain the bracket (116) against the one or more surfaces (104).
Example 12. The protective assembly (100) of any of Examples 1 to 11, wherein the first color is selected to substantially match a color of an interior of an aircraft (150).
Example 13. The protective assembly (100) of any of Examples 1 to 12, wherein the one or more surfaces (104) to which the retrofitted protective bumper (102) is attachable comprises one or more surfaces (104) in an aisleway or corner within an aircraft (150).
Example 14. A method (200) comprising:
   exposing (202) a retrofitted protective bumper (102) to ultraviolet (UV) light using a light-emitter device (110) configured to emit the UV light (108), wherein the retrofitted protective bumper (102) is attached to one or more surfaces (104) in an area such that the retrofitted protective bumper (102) covers at least a portion of the one or more surfaces (104), and wherein the retrofitted protective bumper (102) comprises a photochromic plastic material (106) having a first color; and
   in response to the exposing, causing (204) the photochromic plastic material (106) to change from the first color to a second color that is different from the first color, that is visible under ambient lighting conditions, and that visually indicates UV sanitization of the area.
Example 15. The method (200) of Example 14, wherein exposing (202) the retrofitted protective bumper (102) to the UV light (108) comprises exposing the retrofitted protective bumper (102) to UV light (108) having a wavelength within a range of approximately 200 nanometers to approximately 280 nanometers.
Example 16. The method (200) of Example 14 or 15, wherein exposing (202) the retrofitted protective bumper (102) to the UV light (108) comprises exposing, to the UV light (108), a retrofitted protective bumper (102) that is attached to one or more surfaces (104) in an aisleway or corner within an aircraft (150).
Example 17. The method (200) of any of Examples 14 to 16, wherein causing (204) the photochromic plastic material (106) to change from the first color to the second color comprises causing the photochromic plastic material (106) to change from the first color to a second color that is visible under the ambient lighting conditions without a visual aid device.
Example 18. A method (300) comprising:
   mechanically coupling (302), to a bracket (116) attached to one or more surfaces (104) in an area, a retrofitted protective bumper (102), such that the retrofitted protective bumper (102) covers at least a portion of the one or more surfaces (104),
   wherein the retrofitted protective bumper (102) comprises a photochromic plastic material (106) that, when exposed to ultraviolet (UV) light, changes from a first color to a second color that is different from the first color, that is visible under ambient lighting conditions, and that visually indicates UV sanitization of the area.
Example 19. The method (300) of Example 18, wherein the retrofitted protective bumper (102) comprises an insert (114) having an elongated arcuate base (118) and a first pair of legs (120) protruding from the elongated arcuate base (118), defining a first channel (122) therebetween,
   wherein the bracket (116) comprises an elongated base (124) and a second pair of legs (126) protruding from the elongated base (124) and defining a second channel (128) therebetween, and
   wherein mechanically coupling (302) the retrofitted protective bumper (102) to the bracket (116) comprises mechanically coupling the insert (114) to the bracket (116) using snap-fit insert (114)ion of the insert (114) into the second channel (128) of the bracket (116) such that the first pair of legs (120) interlock with the second pair of legs (126).
Example 20. The method (300) of Examples 18 or 19, wherein mechanically coupling (302) the retrofitted protective bumper (102) to the bracket (116) attached to the one or more surfaces (104) comprises mechanically coupling the retrofitted protective bumper (102) to the bracket (116) attached to one or more surfaces (104) in an aisleway or corner within an aircraft (150).
Example 21. An ultraviolet (UV) sanitization kit (112) comprising:
   a light-emitter device (110) configured to emit UV light (108); and
   a retrofitted protective bumper (102), wherein the retrofitted protective bumper (102) is an insert (114) configured to mechanically couple to a bracket (116) attached to one or more surfaces (104) in an area such that the retrofitted protective bumper (102) covers at least a portion of the one or more surfaces (104),
   wherein the retrofitted protective bumper (102) comprises a photochromic plastic material (106) that, when exposed to UV light (108), changes from a first color to a second color that is different from the first color and that is visible under ambient lighting conditions, thereby visually indicating UV sanitization of the area.
Example 22. The UV sanitization kit (112) of Example 21, wherein the insert (114) comprises an elongated arcuate base (118) and a first pair of legs (120) protruding from the elongated arcuate base (118) and defining a first channel (122) therebetween,
   wherein the bracket (116) comprises an elongated base (124) and a second pair of legs (126) protruding from the elongated base (124) defining a second channel (128) therebetween, and
   wherein the insert (114) is configured to mechanically couple to the bracket (116) by snap-fit insert (114)ion of the insert (114) into the second channel (128) of the bracket (116) such that the first pair of legs (120) interlock with the second pair of legs (126).
Example 23. A protective assembly (100) for visually indicating ultraviolet (UV) sanitization of an area, the protective assembly (100) comprising:
   a retrofitted protective bumper (102) attachable to one or more surfaces (104) in the area such that the retrofitted protective bumper (102) covers at least a portion of the one or more surfaces (104),
   wherein the retrofitted protective bumper (102) comprises a photochromic plastic material (106) that, when exposed to UV light (108), changes from a first color to a second color that is different from the first color, the photochromic plastic material (106) comprising:
      one or more photochromic polymers, present in a combined amount of at least 5 wt.%; and
      at least one of:
         a colloidal metal, or
         a non-photochromic polymer.
Example 24. The protective assembly (100) of Example 23, wherein the photochromic plastic material (106) is substantially homogeneous.

The description of the different advantageous arrangements has been presented for purposes of illustration and description, and is not intended to be exhaustive or limited to the examples in the form disclosed. Many modifications and variations will be apparent to those of ordinary skill in the art. Further, different advantageous examples may describe different advantages as compared to other advantageous examples. The example or examples selected are chosen and described in order to best explain the principles of the examples, the practical application, and to enable others of ordinary skill in the art to understand the disclosure for various examples with various modifications as are suited to the particular use contemplated.

## Claims

1. A protective assembly (100) for visually indicating ultraviolet (UV) sanitization of an area, the protective assembly (100) comprising:
a retrofitted protective bumper (102) attachable to one or more surfaces (104) in the area such that the retrofitted protective bumper (102) covers at least a portion of the one or more surfaces (104),
wherein the retrofitted protective bumper (102) comprises a photochromic plastic material (106) that, when exposed to UV light (108), changes from a first color to a second color that is different from the first color, the photochromic plastic material (106) comprising:
one or more photochromic polymers, present in a combined amount of at least 5 wt.%; and
at least one of:
a colloidal metal, or
a non-photochromic polymer.

2. The protective assembly (100) of claim 1, wherein the photochromic plastic material (106) is substantially homogeneous.

3. An ultraviolet (UV) sanitization kit (112) comprising:
a light-emitter device (110) configured to emit UV light (108); and
a retrofitted protective bumper (102), wherein the retrofitted protective bumper (102) is an insert (114) configured to mechanically couple to a bracket (116) attached to one or more surfaces (104) in an area such that the retrofitted protective bumper (102) covers at least a portion of the one or more surfaces (104),
wherein the retrofitted protective bumper (102) comprises a photochromic plastic material (106) that, when exposed to UV light (108), changes from a first color to a second color that is different from the first color and that is visible under ambient lighting conditions, thereby visually indicating UV sanitization of the area.

4. The UV sanitization kit (112) of claim 3, wherein the insert (114) comprises an elongated arcuate base (118) and a first pair of legs (120) protruding from the elongated arcuate base (118) and defining a first channel (122) therebetween,
wherein the bracket (116) comprises an elongated base (124) and a second pair of legs (126) protruding from the elongated base (124) defining a second channel (128) therebetween, and
wherein the insert (114) is configured to mechanically couple to the bracket (116) by snap-fit insertion of the insert (114) into the second channel (128) of the bracket (116) such that the first pair of legs (120) interlock with the second pair of legs (126).

5. A method (300) comprising:
mechanically coupling (302), to a bracket (116) attached to one or more surfaces (104) in an area, a retrofitted protective bumper (102), such that the retrofitted protective bumper (102) covers at least a portion of the one or more surfaces (104),
wherein the retrofitted protective bumper (102) comprises a photochromic plastic material (106) that, when exposed to ultraviolet (UV) light, changes from a first color to a second color that is different from the first color, that is visible under ambient lighting conditions, and that visually indicates UV sanitization of the area.

6. The method (300) of claim 5, wherein the retrofitted protective bumper (102) comprises an insert (114) having an elongated arcuate base (118) and a first pair of legs (120) protruding from the elongated arcuate base (118), defining a first channel (122) therebetween,
wherein the bracket (116) comprises an elongated base (124) and a second pair of legs (126) protruding from the elongated base (124) and defining a second channel (128) therebetween, and
wherein mechanically coupling (302) the retrofitted protective bumper (102) to the bracket (116) comprises mechanically coupling the insert (114) to the bracket (116) using snap-fit insertion of the insert (114) into the second channel (128) of the bracket (116) such that the first pair of legs (120) interlock with the second pair of legs (126).

7. The method (300) of claim 5 or 6, wherein mechanically coupling (302) the retrofitted protective bumper (102) to the bracket (116) attached to the one or more surfaces (104) comprises mechanically coupling the retrofitted protective bumper (102) to the bracket (116) attached to one or more surfaces (104) in an aisleway or corner within an aircraft (150).
